Europäisches Patentamt

European Patent Office

Office européen des brevets

(19)

(11) Publication number : **0 316 291 B1**

(12) **EUROPEAN PATENT SPECIFICATION**

(45) Date of publication of patent specification :
04.03.92 Bulletin 92/10

(51) Int. Cl.⁵ : **A41D 13/00, A62B 17/04**

(21) Application number : **88830481.3**

(22) Date of filing : **10.11.88**

(54) A face protecting mask intended to be used in general medicine and more particularly in surgery.

(30) Priority : **11.11.87 IT 3610487 U**
**09.11.88 IT 4854188**

(43) Date of publication of application :
**17.05.89 Bulletin 89/20**

(45) Publication of the grant of the patent :
**04.03.92 Bulletin 92/10**

(84) Designated Contracting States :
**AT BE CH DE ES FR GB GR LI LU NL SE**

(56) References cited :
**BE-A- 486 510**
**DE-C- 636 426**
**FR-A- 873 113**

(56) References cited :
**FR-A- 2 309 167**
**US-A- 2 855 924**
**US-A- 3 885 558**
**US-A- 3 955 570**
**US-A- 4 296 746**

(73) Proprietor : **Millauro, Carlo**
**Via A. Richelmy, 38**
**I-00165 Roma (IT)**

(72) Inventor : **Millauro, Carlo**
**Via A. Richelmy, 38**
**I-00165 Roma (IT)**

(74) Representative : **Massari, Marcello**
**Studio M. Massari S.r.l. 23, Via Fontanella Borghese**
**I-00186 Roma (IT)**

EP 0 316 291 B1

## Description

This invention refers to a face protecting mask intended to be used in general medicine and more particularly in surgery comprising: a shell or top portion provided with an elastic band fitted in the rear portion of the trim thereof; a downward front shield provided with an eye-window covered by a strip of a transparent flexible material and a mouth-opening placed below the eye-window and closed by a wad of a filtering material; two side protection flaps connected to the front shield; two pairs of strings for tying the mask; a means from preventing mist from forming on the transparent window and a means for keeping the front shield away from the operator's face.

In the medical field, the present hygienic and sanitary situation made the use of protective elements even more necessary to the operators.

Thus, particularly in the surgical field, the protection offered by the masks used at present that cover only the mouth, the lower portion of the nose and the surrounding areas has become insufficient.

Accordingly, new protection masks should be provided offering a nearly total protection of the operator's head and neck without causing any secondary drawbacks due to the integral structure thereof.

In this field some masks are already known as, for instance, the subject matter of US-A-4,296,746 of Mason.

This surgical mask essentially comprises a unitary rigid shell of transparent plastic material provided with seal means, isolating the lower mask portion, from the upper portion and vent means for allowing air circulation and heat dissipation.

This mask seems to be rather bulky and space wasting both for shipping and storing.

Also the surgical mask subject matter of US-A-3,955,570 has a rather bulky construction essentially comprising a support member and a transparent face plate consisting of a rigid plastic almost spherically shaped, these two members being covered by a third component consisting of a hood of flexible material.

Also this construction, besides to be bulky and space wasting for shipping and storing, is unduly complicate and very expensive.

Finally BE-A-436510 discloses a face mask not specifically intended to be used in the performance of surgical operations.

This mask is mainly a face covering mask intended to fix, over the mouth and nose area of the face, a pad apted to filter the breathed air in presence of poison gas, air pollution and the like.

The construction of this mask is very simple and deprived of any means for preventing fogging of the transparent window and allowing air circulation.

In view of the foregoing, it is an aim of the invention to provide an integrally formed mask offering a complete protection of the upper, front and side portions of the head comprising the ears and the nick which can be comfortably used also in directly or indirectly highly heated rooms, such as operating rooms.

The mask of the invention will be now described in detail with reference to the annexed drawings, wherein:

Figure 1 is a front view of the mask;

Figure 2 is a side view;

Figure 3 is a rear perspective view;

Figure 4 is a sectional view taken on line IV-IV of Figure 3;

Figure 5 is a sectional view taken on line V-V of Figure 4;

Figure 6 is an enlarged view of a detail of Figure 4; and

Figure 7 is an enlarged view of a detail of Figure 1.

As shown in the figures, the mask referred to by reference 10 substantially comprises a top shell 11 and a front shield 12 downwardly extending therefrom having two side flaps 13 for protecting the sides of the operator's head, the upper portion of flaps 13 extending rearwardly and joining to shell 11, thus forming an integral piece.

At the level of the operator's eyes shield 12 has a rectangular opening 14 protected by a band of a flexible transparent material, thus forming a "window" allowing the operator to see.

A similar rectangular opening 16 is formed immediately below window 15 at the level of the operator's mouth, which opening 16 is closed by a "wad" 18 of material filtering the air breathed by the operator.

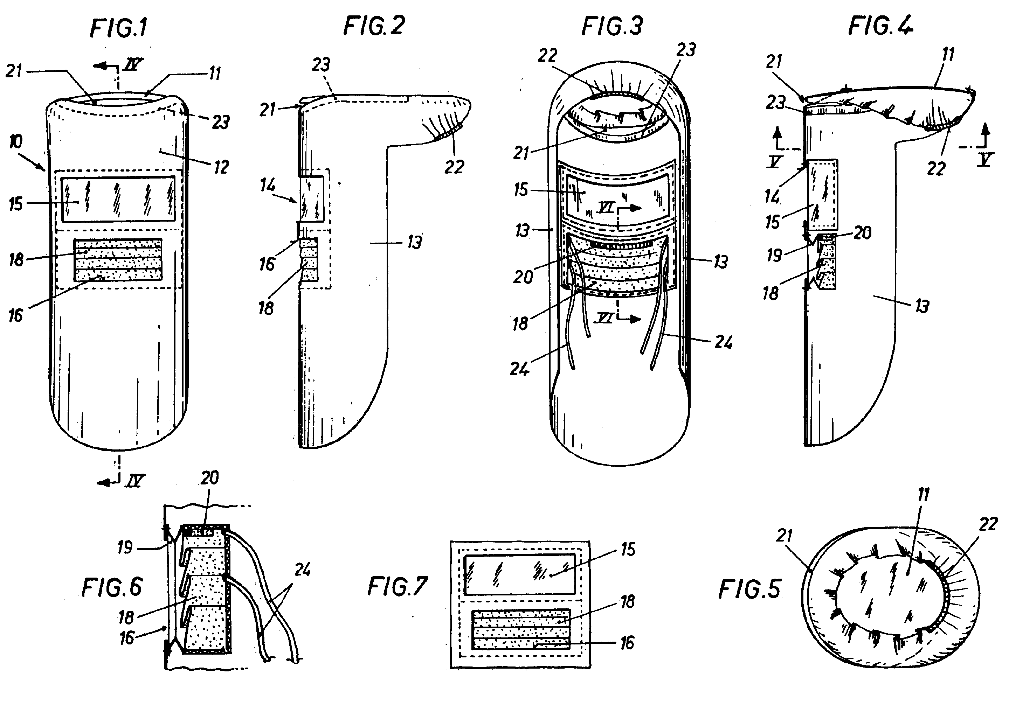

It should be noted that the whole edge of filtering wad 18 is connected to the edge of opening 16 by a "folding" frame 19, as clearly shown in Figures 4 and 6. Furthermore, a listel of a soft foamed material referred to by 20 is attached to the upper edge of wad 18,

As better shown in Figures 1, 2 and 3, the central portion of the upper edge of front shield 12 is not joined to the corresponding length of the upper edge of shell 11 which is bent, thus forming a small opening or slot 21 in this area.

Furthermore, a piece of elastic band referred to by reference 22 is fitted in the rear portion of top shell 11, the elastic band allowing the shell to tighten on the operator's head.

Finally, a substantially semicircular strip 23 of a simirigid material is joined to the upper edge of front shield 12 at the junction thereof with the top of shell 11.

Mask 10 is completed by two pairs of strings 24, one at each side, attached at the sides of filter 18 which closes opening 16.

It should be noted that the mask of the invention having the above-described and illustrated configuration offers a nearly complete protection to the operator's face, head and neck assuring a maximum hygiene both to the patient and to the operator.

The particular and unique comfort offered by the mask of the invention is due to the simultaneous presence of listel 20 placed at the top of filter 18 and opening 21 on one hand and of strip 23 on the other hand.

Listel 20, which is in contact with the operator's face prevents the vapour contained in the expelled air from condensing on transparent window 15, while the opening helps the air circulation in the upper portion of the mask and is essential in preventing also the vapours due to perspiration from condensing on window 15.

Semicircular strip 23 keeps front shield 12 away from the operator's face and this is particularly convenient in case the operator wears spectacles.

The further advantage due to folding frame 19 should also be noted, which frame allows any movement of filter 18 without causing any movements of transparent window 15 that could be uncomfortable.

The materials used will preferably be viscose not woven tissue containing polypropylene for shell 11, shield 12 and flaps 13 and a similar material having a higher density for filter 18.

Finally, transparent protection 15 of window 14 will preferably be of polyester.

Listel 20 and strip 23 will preferably be provided with a self-adhesive material allowing the same to be easily placed in position on the mesk.

**Claims**

1. A surgical mask for protecting the operator's head consisting of a top shell (11) and front shield (12) downwardly extending therefrom having two side flaps (13) for protecting the sides of operator's head, all of a non-woven tissue, a transparent window (15) and an opening (16) closed by a "wad" (18) of a material filtering the air, placed below said window (15), means for preventing mist from forming on said window, means for helping air circulation within said mask, characterized in that said filter (18) is mounted on said opening (16) through a folding frame (19) allowing any movement of said filter (18) without causing any movement of said transparent window (15).

2. The protection mask according to claim 1, wherein said means for helping air circulation within said mask comprises a slot (21) formed at the junction of said shield (12) with said shell (11).

3. The protection mask according to claim 1, further provided with means for keeping said shield (12) away from the operator's face comprising a semicircular strip (23) of a semirigid material connected to the upper edge of said front shield (12).

**Patentansprüche**

1. Operationsmaske zum Schutz des Kopfes des Operateurs, bestehend aus einer oberen Schale (11) und einem davon nach unten verlaufenden vorderen Schild (12) mit zwei Seitenklappen (13) zum Schutz der Kopfseiten des Operateurs, sämtlich aus Vliesstoff, einem durchsichtigen Fenster (15) und einer Öffnung (16), die über einen unterhalb des Fensters (15) angeordneten "Bausch" (18) aus einem die Luft filternden material verschlossen ist, einer Einrichtung, die ein Beschlagen des Fensters verhindert, einer Einrichtung zur Unterstützung der Luftzirkulation in der maske, dadurch gekennzeichnet, daß der Filter (18) über einen Faltrahmen (19), der eine Bewegung des Filters (18) zuläßt, ohne eine Bewegung des durchsichtigen Fensters (15) zu bewirken, auf der Öffnung (16) angeordnet ist.

2. Schutzmaske nach Anspruch 1, wobei die Einrichtung zur Unterstützung der Luftzirkulation in der maske einen Schlitz (21) aufweist, der an der Verbindungsstelle des Schilds (12) mit der Schale (11) gebildet ist.

3. Schutzmaske nach Anspruch 1, die ferner eine Einrichtung umfaßt, die den Schild (12) vom Gesicht des Operateurs entfernt hält und einen halbkreisförmigen Streifen (23) aus einem halbsteifen material aufweist, der mit dem oberen Rand des vorderen Schilds (12) verbunden ist.

**Revendications**

1. Masque chirurgical destiné à protéger la tête de l'opérateur, constitué d'une coquille supérieure (11) et d'un écran de protection avant (12) partant vers le bas de la coquille et comportant deux pans latéraux (13) destinés à protéger les côtés de la tête de l'opérateur, tous ces éléments étant en tissu non tissé, une fenêtre transparente (15) et une ouverture (16) fermée par un "tampon" (18) d'un matériau de filtrage de l'air, cette ouverture étant placée au-dessous de la fenêtre (15), des moyens destinés à empêcher la formation de buée sur la fenêtre, et des moyens destinés à faciliter la circulation d'air à l'intérieur du masque, masque caractérisé en ce que le filtre (18) est monté sur l'ouverture (16) par l'intermédiaire d'un châssis pliant (19) permettant au filtre (18) d'effectuer un mouvement quelconque sans produire le moindre mouvement de la fenêtre transparente (15).

2. Masque de protection selon la revendication 1, caractérisé en ce que les moyens destinés à faciliter la circulation d'air à l'intérieur du masque comprennent une fente (21) formée à la jonction de l'écran de protection (12) avec la coquille (11).

3. Masque de protection selon la revendication 1, caractérisé en ce qu'il comprend en outre des moyens destinés à maintenir l'écran de protection (12) écarté de la figure de l'opérateur, ces moyens comprenant une bande semi-circulaire (23) d'un matériau semi-rigide relié au bord supérieur de l'écran de protection avant (12).

FIG.1
FIG.2
FIG.3
FIG.4
FIG.5
FIG.6
FIG.7